# EUROPÄISCHE PATENTANMELDUNG

(11) **EP 0 702 023 A1**
(43) Veröffentlichungstag der Anmeldung: **20.03.1996**
(21) Anmeldenummer: 95110919.8
(22) Anmeldetag: 12.07.1995
(51) Int. Cl.: C07H 15/04

(54) **Verfahren zur Herstellung von Alkylglycosiden mit niedrigem Glycosidierungsgrad**

(30) Priorität: 07.09.1994 DE 4431855
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Nach dem vorliegenden Verfahren werden Alkylglycoside mit C₈- bis C₂₀-Alkylresten, mit einem mittleren Glycosidierungsgrad von 1,05 bis 1,4 und einem geringen Polysaccharidgehalt dadurch hergestellt, daß man ein Monosaccharid mit einem Dextroseäquivalent von 96 bis 99,5 einsetzt und bei der Glycosidierung ein Monosaccharid-Alkohol-Molverhältnis von 1 : 5 bis 1 : 20 sowie bei der Umglycosidierung ein Alkylglycosid-Fettalkohol-Molverhältnis von 1 : 2 bis 1 : 10 einstellt.

## Beschreibung

Die Erfindung betrifft ein neues Verfahren zur Herstellung von Alkylglycosiden durch Glycosidierung und Umglycosidierung, wobei die Alkylglycoside C₈- bis C₂₀-Alkylreste und einen mittleren Glycosidierungsgrad von 1,05 bis 1,4 und einen Polysaccharidgehalt von bis zu 10 % aufweisen.

Alkylglycoside mit C₈- bis C₂₀-Alkylgruppen können ganz oder teilweise aus nachwachsenden Rohstoffen hergestellt werden. Sie gewinnen deshalb und auch wegen ihrer sehr guten biologischen Abbaubarkeit zunehmend an Bedeutung. Die Produkte haben neben ihren interessanten Tensideigenschaften den Vorteil, daß man ihre Polarität über die Länge der Alkylkette und über den Glycosidierungsgrad genau einstellen kann. Dadurch können die Alkylglycoside auf ihr Anwendungsgebiet gezielt ausgerichtet werden.

Bei der zweistufigen Alkylglycosidherstellung wird zunächst eine Glycosidierung durchgeführt. Aus Sacchariden und C₂- bis C₆-Alkoholen werden dabei Alkylglycoside mit kurzkettigen Alkylgruppen hergestellt. Diese Produkte werden dann in der zweiten Stufe durch Umglycosidierung mit C₈- bis C₂₀-Alkoholen in die gewünschten Alkylglycoside mit Tensideigenschaften übergeführt.

Dieser Weg der Herstellung ist lange bekannt. Neuere Anmeldungen auf diesem Gebiet befassen sich häufig mit der Herstellung farblich verbesserter Produkte. Dabei werden zum Teil Reduktionsmittel zugesetzt oder spezielle Apparaturen verwendet.

So wird in EP-A-0 501 032 die Glycosidierung in einem Verdampfer vorgenommen. Der dort vorzugsweise verwendete Glucosesirup ist im allgemeinen ein Stärkehydrolysat mit Oligosaccharidanteilen. Das Dextroseäquivalent derartiger Sirupe liegt meist bei 90 bis 95. Die hier hergestellten Alkylpolyglycoside mit langkettigen Alkylgruppen weisen vorzugsweise mittlere Glycosidierungsgrade im Bereich von 1,3 bis 5 auf, wobei im Beispiel ein Wert von 1,65 angegeben wird.

Nach EP-A-0 514 628 kann auch die Umglycosidierung in einem Verdampfer vorgenommen werden. In EP-A-0 514 627 wird die Umglycosidierung in einer Reaktionskolonne durchgeführt. Dabei stellt man ein Molverhältnis von Alkylglycosid mit kurzem Alkylrest zu langkettigem Alkohol von 1 : 2 bis 1 : 15 ein und erhält Alkylpolyglycoside mit Glycosidierungsgraden von vorzugsweise 1,2 bis 3.

Zur destillativen Abtrennung der Fettalkohole von Alkylpolyglycosiden mit C₁₀- bis C₁₈-Alkylgruppen und einem mittleren Glycosidierungsgrad von 1,05 bis 1,4 verwendet man gemäß DE-A-41 29 587 einen Dünnschichtverdampfer. Auf die Herstellung der Alkylpolyglycoside wird hier nicht näher eingegangen.

WO 93/101 33 beschreibt ein zweistufiges Verfahren zur Herstellung von Alkyloligoglycosiden, das kontinuierlich oder diskontinuierlich durchgeführt werden kann und bei dem ein Glucosesirup mit einem Anteil an monomerer Glucose von 90 bis 100 % eingesetzt wird. Das hier beschriebene Verfahren ist insgesamt recht aufwendig. Es erfordert sowohl bei der Glycosidierung als auch bei der Umglycosidierung eine Nachreaktion. Außerdem müssen in beiden Reaktionsstufen enge Temperaturbereiche eingehalten werden. WO 93/101 33 liefert dabei Alkyloligoglucoside mit einem Polyglucosegehalt von mindestens 4,7 Gewichtsprozent. Zum mittleren Glucosidierungsgrad werden keine Angaben gemacht.

Einen Hinweis darauf, wie Alkylglycoside mit mittleren Glycosidierungsgraden von 1,05 bis 1,4 und gleichzeitig niedrigen Gehalten an Polysaccharid in einfacher Weise hergestellt werden können, liefern die genannten Schriften nicht.

Es war daher Aufgabe der vorliegenden Erfindung, ein einfaches zweistufiges Verfahren zur Herstellung von Alkylglycosiden mit C₈- bis C₂₀-Alkylresten und mit einem mittleren Glycosidierungsgrad von 1,05 bis 1,4 und einem niedrigen Polysaccharidgehalt bereitzustellen. Die Produkte sollten darüber hinaus auch eine gute Farbqualität aufweisen.

Die Aufgabe wird erfindungsgemäß dadurch gelöst, daß man ein Monosaccharid mit einem Dextroseäquivalent von 96 bis 99,5 einsetzt und außerdem bei der Glycosidierung ein Molverhältnis von Monosaccharid zu C₂- bis C₆-Alkohol von 1 : 5 bis 1 : 20 und bei der Umglycosidierung ein Molverhältnis von Alkylglycosid mit C₂- bis C₆-Alkylrest zu C₈- bis C₂₀-Fettalkohol von 1 : 2 bis 1 : 10 einstellt.

Überraschenderweise gelingt es durch diese Maßnahmen, ohne Filtrationsschritte beispielsweise in einfachen Rührkesselkaskaden farblich ansprechende Produkte mit niedrigem Glycosidierungsgrad und niedrigem Polysaccharidgehalt herzustellen.

Für das Verfahren einsetzbare Monosaccharide sind beispielsweise Glucose, Mannose, Galaktose, Gulose, Allose oder Talose. Vorzugsweise wird dabei Glucose verwendet. Die Monosaccharide können dabei auch in der Form eines wäßrigen Sirups, der bis zu 50 % Wasser enthalten kann, eingesetzt werden.

Zur Charakterisierung der Monosaccharide wird der Gehalt an reduzierenden Zuckern, berechnet als Dextrose auf der Basis der Trockensubstanz, bestimmt und als Dextroseäquivalent bezeichnet.
Während demnach eine reine Hexopyranose, wie Glucose, ein Dextroseäquivalent von 100 aufweist, zeigen Dextroseäquivalente unter 100 einen Anteil an Oligosacchariden an.
Erfindungsgemäß liegt das Dextroseäquivalent vorzugsweise bei 96 bis 99.

Als zur Glycosidierung einsetzbare Alkohole sind beispielsweise Ethanol, n-Propanol, Isopropanol, n-Butanol, t-Butanol, Amylalkohol oder Hexanol geeignet.

Monosaccharid und C₂- bis C₆-Alkohol werden vorzugsweise im molaren Verhältnis von 1 : 5 bis 1 : 10 zur Reaktion gebracht.

Die bei der Glycosidierung hergestellten Alkylglycoside mit C₂- bis C₆-Alkylketten werden in einer zweiten Stufe, der Umglycosidierung, mit C₈- bis C₂₀-Fettalkoholen umgesetzt. Die Fettalkohole können dabei linear oder verzweigt sein. Sie können auch olefinische Doppelbindungen enthalten. Man kann natürliche oder synthetische Fettalkohole oder Fettalkoholgemische einsetzen. Als Beispiele seien Octanol, Decanol, 10-Undecen-1-ol, Dodecanol, Myristylalkohol und Stearylalkohol genannt. Vorzugsweise werden Fettalkohole mit 10 bis 14 C-Atomen eingesetzt. Meist wird das Alkylglycosid mit C₂- bis C₆-Alkylgruppen im Molverhältnis von 1 : 4 bis 1 : 10 mit dem Fettalkohol umgesetzt.

Bei der Glycosidierung und Umglycosidierung kann man als saure Katalysatoren Mineralsäuren, wie Schwefel- oder Salzsäure, einsetzen. Gut geeignet sind auch organische Säuren, wie beispielsweise Aryl-, Alkyl- oder Aralkylsulfonsäuren. Dabei liegt die Reaktionstemperatur im allgemeinen im Bereich von 100 bis 140 °C. Die Glycosidierung führt man vorzugsweise bei 100 bis 125 °C durch. Bei der Umglycosidierung liegt die Reaktionstemperatur meist bei 105 bis 135 °C, wobei zur Beschleunigung der Reaktion, d. h. zur Abtrennung des kurzkettigen Alkohols, meist Vakuum angelegt wird.

Die zweistufige Herstellung der Alkylglycoside kann kontinuierlich oder diskontinuierlich erfolgen. Dabei wird die kontinuierliche Alkylglycosid-Synthese üblicherweise in einer Rührkesselkaskade durchgeführt, wobei die Rührkessel auch teilweise oder vollständig durch Reaktionskolonnen oder Rohrreaktoren ersetzt werden können.

Das Produkt der Umglycosidierung wird in bekannter Art und Weise mit geeigneten Basen neutralisiert. Der überschüssige Fettalkohol wird dann destillativ abgetrennt, was in einem Dünnschicht- oder Kurzwegverdampfer erfolgen kann. Das gewonnene Alkylglycosid, das vorzugsweise einen mittleren Glycosidierungsgrad von 1,1 bis 1,3 aufweist, wird dann mit Wasser abgemischt und mit Peroxid gebleicht. Das Endprodukt weist eine Iodfarbzahl (IFZ) von unter 20 und vorzugsweise einen Restfettalkoholgehalt von unter 1 % auf.

Die Produkte zeichnen sich darüber hinaus durch einen niedrigen Polysaccharidgehalt von bis zu 10 % aus. Dieser Gehalt bezieht sich dabei auf den Feststoff, also auf die Summe von Alkylglycosid und Polysaccharid. Der Polysaccharidgehalt liegt vorzugsweise bei 1 bis 10 %.

Das erfindungsgemäße Verfahren führt bei Einsatz von Monosacchariden der genannten Qualität und bei Einhaltung der beanspruchten Molverhältnisse zu Reaktionsmischungen, die wegen des niedrigen Polysaccharidgehalts einen sehr geringen Anteil an ungelösten Bestandteilen aufweisen. Rohrleitungen und Reaktoren neigen deshalb nicht zu Verstopfungen. Das erhöht die Betriebssicherheit. Gleichzeitig werden direkt Alkylglycoside mit geringem Glycosidierungsgrad erhalten, wie sie mit Vorteilen in Wasch- und Geschirrspülmitteln eingesetzt werden.

Die folgenden Beispiele sollen die Erfindung verdeutlichen.

### Vergleichsbeispiel A

Eine Kaskade aus zwei 4-l-Rührreaktoren wird stündlich mit 1,5 l n-Butanol, 100 ml butanolischer p-Toluolsulfonsäurelösung (173 g/l p-Toluolsulfonsäure) und 540 ml 70%iger Glucoselösung gespeist. Die verwendete Glucose hat ein Dextroseäquivalent von 95. Über aufgesetzte Kolonnen wird bei 120 °C Innentemperatur Wasser azeotrop abdestilliert. Das dabei ebenfalls destillierte n-Butanol wird nach Abtrennung der Wasserphase in die Kolonne zurückgeführt. Den zweiten Rührreaktor verlassen stündlich ca. 1 850 ml Lösung aus ca. 65 % Butanol und 35 % Butylglucosid.

Dieses Produkt wird mit einem Mengenstrom von 4 l Fettalkohol (Gemisch aus 68 % Dodecanol, 26 % Tetradecanol und 6 % Hexadecanol) vermischt und dann durch eine weitere Kaskade aus zwei 4-l-Rührreaktoren geleitet. Bei einer Reaktionstemperatur von 120 °C und 45 mbar (erster Reaktor) bzw. 5 mbar (zweiter Reaktor) wird über eine aufgesetzte Kolonne Butanol abdestilliert.

Das Produkt, eine ca. 25%ige fettalkoholische Alkylglucosid-Lösung, wird mit 25%iger NaOH auf pH 10 eingestellt (gemessen in einem 1 : 1 : 1-Gemisch aus Produktlösung, Isopropanol und Wasser). Der Fettalkohol wird anschließend in einer Kaskade von Dünnschichtverdampfern bis auf einen Restgehalt von ca. 1 % im Vakuum abdestilliert. Das erhaltene Alkylglucosid weist einen mittleren Glycosidierungsgrad von 1,44 und einen Polyglucosegehalt von 13,5 % auf.

### Beispiel 1

Es wird wie im Vergleichsbeispiel A verfahren. Man verwendet jedoch Glucose mit einem Dextroseäquivalent von 99. In diesem Falle erhält man ein Alkylglycosid mit einem mittleren Glycosidierungsgrad von 1,16 und einem Polyglucosegehalt von 6,9 %.

## Patentansprüche

1. Verfahren zur Herstellung von Alkylglycosiden mit C₈- bis C₂₀-Alkylresten, einem mittleren Glycosidierungsgrad von 1,05 bis 1,4 und einem Polysaccharidgehalt von bis zu 10 % durch Glycosidierung und Umglycosidierung,
dadurch gekennzeichnet,
daß man
- ein Monosaccharid mit einem Dextroseäquivalent von 96 bis 99,5 einsetzt,
- das Monosaccharid bei der Glycosidierung im Molverhältnis 1 : 5 bis 1 : 20 mit einem C₂- bis C₆-Alkohol und
- das dabei erhaltene Alkylglycosid mit einem C₂- bis C₆-Alkylrest zwecks Umglycosidierung im Molverhältnis 1 : 2 bis 1 : 10 mit einem C₈- bis C₂₀-Fettalkohol umsetzt.

2. Verfahren nach Anspruch 1,
dadurch gekennzeichnet,
daß der mittlere Glycosidierungsgrad bei 1,1 bis 1,3 liegt.

3. Verfahren nach Anspruch 1,
dadurch gekennzeichnet,
daß der Polysaccharidgehalt bei 1 bis 10 % liegt.

4. Verfahren nach Anspruch 1,
dadurch gekennzeichnet,
daß Glucose mit einem Dextroseäquivalent von 96 bis 99 eingesetzt wird.

5. Verfahren nach Anspruch 1,
dadurch gekennzeichnet,
daß das Molverhältnis der Glycosidierung 1 : 5 bis 1 : 10 und das der Umglycosidierung 1 : 4 bis 1 : 10 beträgt.

6. Verfahren nach Anspruch 1,
dadurch gekennzeichnet,
daß man C₁₀- bis C₁₄-Fettalkohole verwendet.
